Europäisches Patentamt

European Patent Office

Office européen des brevets

(19)

(11) Veröffentlichungsnummer: **0 155 684**
**B1**

(12) **EUROPÄISCHE PATENTSCHRIFT**

(45) Veröffentlichungstag der Patentschrift: **24.10.90**

(51) Int. Cl.⁵: **A 61 M 1/36, B 04 B 5/04**

(21) Anmeldenummer: **85103248.2**

(22) Anmeldetag: **20.03.85**

(54) **Vorrichtung zur Trennung von Blut.**

(30) Priorität: **21.03.84 DE 3410286**

(43) Veröffentlichungstag der Anmeldung:
**25.09.85 Patentblatt 85/39**

(45) Bekanntmachung des Hinweises auf die
Patenterteilung:
**24.10.90 Patentblatt 90/43**

(84) Benannte Vertragsstaaten:
**AT CH DE FR GB IT LI**

(56) Entgegenhaltungen:
**DE-A-2 617 824**
**US-A-3 489 145**
**US-A-4 187 979**

(73) Patentinhaber: **Fresenius AG**
**Gluckensteinweg 5**
**D-6380 Bad Homburg v.d.H. (DE)**

(72) Erfinder: **Neumann, Hans-Jürgen, Dr.**
**Dillingerstrasse 13**
**D-6690 St. Wendel (DE)**
Erfinder: **Meisberger, Artur**
**Winterbacherstrasse 61**
**D-6690 St. Wendel (DE)**
Erfinder: **Weber, Wolfram**
**Albert-Schweizer-Strasse 33**
**D-6683 Spiesen (DE)**

(74) Vertreter: **Dr. Fuchs, Dr. Luderschmidt Dipl.-**
**Phys. Seids, Dr. Mehler Patentanwälte**
**Abraham-Lincoln-Strasse 7**
**D-6200 Wiesbaden (DE)**

**Beschreibung**

Die Erfindung betrifft eine Vorrichtung zur Trennung von Blut, insbesondere zur Gewinnung von Thrombozyten, Leukozyten und/oder Plasma, nach den Merkmalen des Oberbegriffs des Hauptanspruchs, wobei das vom Spender kommende Vollblut in einer Separationseinrichtung einer Zentrifuge in eine Erythrozytenfraktion, zellarmes Plasma, eine Thrombozytenfraktion und/oder gegebenenfalls eine Leukozytenfraktion getrennt wird, die abgetrennten Fraktionen des Blutes über ein Schlauchsystem von Abführungsleitungen aus der Separationskammer der Zentrifuge ausgetragen werden und abgetrenntes zellarmes Plasma teilweise zurückgeführt wird.

Es sind bereits verschiedene Verfahren und Vorrichtungen zur Trennung von Vollblut in seine verschiedenen Komponenten bekannt, wobei eine in-vivo-Blutbehandlung vorgenommen wird, indem einem Spender bzw. Patienten Blut entnommen wird, dieses in einer Separationsvorrichtung einer Zentrifuge in alle oder einige seiner Komponenten, d.h. Erythrozyten, Leukocyten, Thrombozyten und Plasma aufgetrennt wird und eine oder mehrere zu gewinnende Komponenten aus dem System entfernt werden, während die verbleibende Blutfüssigkeit zum Spender bzw. Patienten zurückgeführt wird. Beispiele für solche Verfahren und Vorrichtungen zur Durchführung derselben sind in der DE—A—28 45 399, DE—A—28 45 364, DE—A—28 21 057, DE—A—26 24 154, DE—A—28 21 055, DE—A—29 25 010, DE—A—23 54 368, DE—A—26 12 988, US—A—39 55 755, US—A—39 57 197, US—A—40 07 871, US—A—40 10 894, US—A—41 46 172, US—A—43 30 080 und US—A—34 89 145 beschrieben.

Bei der Separation von Blutkomponenten mit der Zentrifuge stellen die Thrombozyten- und Leukozytenseparation neben der Plasmaseparation wichtige Anwendungsgebiete dar. Bei der Zellseparation, d.h. Erythrozyten-, Leukozyten-(Granulozyten- und Lymphozyten-) bzw. Thrombozytenseparation, wird der Patient bzw. Spender über ein oder zwei Verbindungen mit der Armvene über mehrere Stunden, typischerweise ein bis drei Stunden, mittels eines Schlauchsystems an den Separator einer Zentrifuge angeschlossen. Die Zellausbeute, d.h. die Gesamtzahl an von der jeweils gewünschten Zellsorte aus dem Blut gesammelten Zellen, ist im wesentlichen durch den maximalen Fluß des Vollblutes, der durch den maximalen Venenfluß des Patienten von typischerweise 50—80 ml/min begrenzt ist, und den Wirkungsgrad des Separators der Zentrifuge limitiert. Alle bisher bekannten Verfahren und marktgängigen Zellseparationseinrichtungen besitzen den Nachteil, daß ihr Wirkungsgrad und damit auch die Zellausbeute gering ist und somit für die gewünschte Zellseparation ein erheblicher Aufwand erforderlich ist und der Patient bzw. Spender in starkem Maß belastet werden muß, um zum gewünschten Ergebnis zu gelangen. Eine negative Belastung ist hierbei einmal in einer langen Behandlungszeit mit gleichzeitiger starker Kreislaufbelastung und zum anderen in einer Schädigung der dem Patienten zurückgeführten Blutkomponenten zu sehen.

Es besteht daher ein dringender Bedarf nach neuen Verfahren und Vorrichtungen mit verbessertem Wirkungsgrad bei der Zellgewinnung und größerer Blutschonung.

Bei den bislang bekannten Verfahren wird eine Erhöhung des Wirkungsgrades lediglich durch optimale Einstellung der bei der Zentrifugation sich einstellenden. Phasengrenzen zwischen einzelnen Zellarten angestrebt. Die Phasengrenzen sollen möglichst optimal zu räumlich fest vorgegebenen Auftrennvorrichtungen für die Fraktionen eingestellt werden. Möglichkeiten hierfür sind eine manuelle Regelung des Verhältnisses Plasmafluß zu Erythrozytenfluß, wie in der US—A—34 89 145 und der PCT/US 81/01096 beschrieben, sowie eine automatische oder halbautomatische Regelung der Plasmapumpe, wie in der US—A—41 46 172, US—A—39 55 755 und der EP—A—116 716 beschrieben.

Auch das Verfahren nach der US—A—39 57 197 ist unter diesem Gesichtspunkt zu sehen: das Verhältnis Plasmafluß zu Erythrozytenfluß wird hier durch eine Regelung des Hämatokritwertes des in die Zentrifuge gelangenden Blutes auf einen konstanten, aber definierten Wert eingestellt.

Das Verfahren gemäß dieser Patentschrift wird daher so durchgeführt, daß mittels kontinuierlicher elektrischer Leitfähigkeitsmessungen der Hämatokritwert des eintretenden Vollblutes in der Beschickungsleitung gemessen wird und der Hämatokritwert des eintretenden Vollblutes durch Hinzufügen einer abgetrennten Fraktion (Plasmafraktion oder Erythrozytenfraktion) aus den entsprechenden Leitungen über eine exakt geregelte Pumpe (Pumpe 7) zur Beschickungsleitung für das Vollblut auf einen ausgewählten Wert reguliert wird.

Die US—A—41 87 979, die den Oberbegriff ist vorliegenden Anspruch 1 bildet, zeigt ein Verfahren und eine Vorrichtung zur fraktionierten Auftrennung von Blut in einzelne Blutbestandteile. Zu diesem Zwecke umfaßt die Vorrichtung drei Separationskammern, welche unabhängig voneinander durchströmbar sind. In der ersten Separationskammer erfolgt eine Zerlegung des Vollblutes in Blutzellen und Plasma, in der zweiten Separationskammer werden Leukozyten von dem Plasma getrennt, während in der dritten Separationskammer Blutblättchen vom Plasma getrennt werden. Das in der ersten Separationskammer gewonnene Plasma wird einem Sammelbehälter zugeführt, wobei in der Zuführleitung eine Pumpe angeordnet ist. Stromab der Pumpe zweigt von der Leitung eine Verzweigungsleitung ab, durch welche wahlweise ein Teil des Plasmas der zweiten Separationskammer zugeführt werden kann.

Aufgabe der vorliegenden Erfindung ist es, eine Vorrichtung bereitzustellen, womit es möglich ist, durch einfache Mittel einen beliebigen, insbesondere niedrigen Hämatokritwert des in die Trennkammer der Zentrifuge eintretenden Vollblutes zu erreichen und damit den Wirkungsgrad bei der Zellgewinnung zu erhöhen und gleichzeitig blutschonend zu arbeiten.

Die Aufgabe wird erfindungsgemäß durch die Merkmalskombination des Hauptanspruchs gelöst, die Unteransprüche zeigen vorteilhafte Weiterbildungen der Erfindung.

Die erfindungsgemäße Vorrichtung zeichnet sich durch eine Reihe erheblicher Vorteile aus, welche insbesondere durch die Beschreibung des unter Verwendung der Vorrichtung durchzuführenden Verfahrens besonders deutlich darstellbar sind. In den folgenden Ausführungen wird deshalb zunächst auf die Beschreibung des Verfahrens eingegangen.

Mit der erfindungsgemäßen Vorrichtung kann das Verfahren so durchgeführt werden, daß das vom Spender bzw. Patienten kommende, gegebenenfalls entsprechend vorbehandelte (d.h. z.B. mit Antikoagulanz versetzte) Vollblut über eine Beschickungs- oder Zuführungsleitung in eine Separationskammer einer Zentrifuge eingeführt wird und dort in einer oder mehreren Separationskammern (je nach Art des angewandten Separators), beispielsweise ein oder zwei Kammern, der Zentrifugalkraft entsprechend dem spezifischen Gewicht der Blutkomponenten in eine Erythrozytenfraktion, zellarmes Plasma, eine Thrombozytenfraktion und/oder gegebenenfalls Leukozytenfraktionen (Granulozyten und Lymphozyten) getrennt wird. Über Abführungsleitungen werden dann die einzelnen Fraktionen getrennt aus dem Separator entfernt, was in üblicher Weise gegebenenfalls unter Anwendung von regelbaren Pumpen, vorzugsweise Schlauchpumpen, erfolgt. Die Entfernung des zellarmen Plasmas aus der Separationskammer erfolgt ebenfalls über eine Abführungsleitung oder einen Abführkanal, wobei zwischen dem Zellseparationsteil der Zentrifuge und vor der zum Ableiten des Plasmas vorgesehenen (regelbaren) Plasma-Förderpumpe eine Ab- oder Verzweigung angeordnet ist, über die ein Teil des zellarmen Plasmas in eine weitere Leitung geführt wird und dort mittels einer konstant betriebenen Pumpe, d.h. konstant bestimmte Mengen fördernden Pumpe, mit konstantem Fluß zu dem Vollblut zurückgeführt wird.

Mit der erfindungsgemäßen Vorrichtung kann das zellarme Plasma, in gleicher Weise wie vorstehend ausgeführt, auch zu den abgetrennten Erythrozyten im Separator zurückgeführt werden.

Unter dem Ausdruck "zellarmes Plasma" wird hierin ein Plasma verstanden, das im Separationsverfahren anfällt und abhängig von der zu gewinnenden Zellsorte, z.B. Leukozyten und/oder Thrombozyten, leukozytenarm, d.h. ein Plasma, das bei der Leukozytengewinnung anfällt und aus dem die Leukozyten abgetrennt sind, oder thrombozytenarm, d.h. ein Plasma, das bei der Thrombozytengewinnung anfällt und aus dem im wesentlichen alle Blutzellen abgetrennt sind, ist.

Die erfindungsgemäße Vorrichtung beruht auf dem Prinzip der Verdünnung.

Das geschieht auf einfache Weise dadurch, daß bei den bekannten Separationseinrichtungen zur Trennung von Blut lediglich eine einfache Zusatzeinrichtung wie beispielsweise ein zusätzlicher Pumpenschlauch, mit einer konstant betriebenen Pumpe angebracht wird. Eine solche Zusatzeinrichtung kann bei allen bekannten Ein- bzw. Vorrichtungen zur Trennung von Blut, d.h. auch solchen, wie sie in den vorstehend zum Stand der Technik genannten Druckschriften beschrieben sind, eingesetzt werden, vorausgesetzt, daß sie das vorstehend genannte Zuführungs- oder Abführungsleitungen umnfassende Schlauchsystem besitzen. Diese Zusatzeinrichtung ist an der Abführungsleitung für das zellarme Plasma aus dem Separator stromabwärts, d.h. ausgangsseitig, angeordnet, und zwar so, daß sie sich in dem Teil der Abführungsleitung, der zwischen dem Separator und der Plasma- oder Plasmaförderpumpe liegt, befindet. Sie kann ein übliches Schlauchstück darstellen, das an einem Ende in üblicher Weise mit der Abführungsleitung für das zellarme Plasma verbunden ist, mit einer konstant betriebenen, vorbestimmte Mengen fördernden Pumpe verbunden ist und am anderen Ende in üblicher Weise mit der Beschickungs- bzw. Zuführungsleitung für das Vollblut verbunden ist. Durch dieses Schlauchstück wird ein Teil des zellarmen Plasmas in konstantem Fluß in das Vollblut zurückgeführt. Es ist jedoch auch möglich, das andere Ende des Schlauchstückes anstelle mit der Zuführungsleitung für das Vollblut mit dem Teil der Separationskammer, in welchem sich die separierten Erythrozyten befinden, zu verbinden und so einen Teil des zellarmen Plasmas in konstantem Fluß zu den im Separator befindlichen Erythrozyten zurückzuführen.

Die erstere der genannten Ausführungen hat den praktischen Vorteil, mit keinerlei Zusatzverbindungen bzw. Schläuchen in die Zentrifuge hinein oder aus der Zentrifuge heraus gehen zu müssen.

Das Material für die Zusatzeinrichtung ist im wesentlichen das gleiche Material, wie es auch für die anderen Zuführungs- bzw. Abführungsleitungen, d.h. die anderen Tiele des Schlauchsystems, eingesetzt wird und ist vorzugsweise aus einem mit Blut verträglichen Kunststoff.

Die erfindungsgemäß angewandte Separationseinrichtung kann eine oder mehrere Separationskammern, beispielsweise eine oder zwei Kammern, aufweisen, die miteinander in üblicher Weise, beispielsweise über Verbindungsleitungen, verbunden sind.

Das Schlauchsystem der erfindungsgemäßen Vorrichtung umfaßt die Zuführungs- bzw. Beschickungsleitung für das Vollblut, die Abführungsleitungen mit regelbaren Pumpen zum Abführen der abgetrennten Blutkomponenten, wie Erythrozyten, Thrombozyten und zellarmes Plasma sowie gegebenenfalls Leukozyten (Granulozyten, Lymphozyten) sowie die Zusatzeinrichtung (Pumpenschlauch, Schlauch und konstant betriebene Pumpe) für die Rückführung des abgetrennten zellarmen Plasmas, deren eines Ende vom Separator stromabwärts mit der Abführungsleitung für das Plasma verbunden ist und deren anderes Ende entweder mit der Zuführungs- oder Beschickungsleitung für das Vollblut oder mit dem Teil des Separators, in dem separierte Erythrozyten vorliegen, verbunden ist. Die Verbindung des

3

Schlauchsystems mit dem Separator einerseits und dem Spender oder Patienten andererseits geschieht in üblicher Weise.

Durch die erfindungsgemäßen Vorrichtung ist es überraschend möglich, auf einfache Weise den Wirkungsgrad des Zellseparators und damit die Zellausbeute um den Faktor von rund 1,5 gegenüber bisher bekannten und marktgängigen Verfharen und Vorrichtungen zu erhöhen, und gleichzeitig alle Blutkomponenten bei der Separation sehr schonend zu behandeln, d.h. die Funktionsfähigkeit so wenig wie nötig zu beeinträchtigen. Ferner wird durch die erfindungsgemäße Verfahrungsweise (Verdünnung) die Vorrichtung weniger empfindlich gemacht.

Obgleich nachfolgend die Vorrichtungen bzw. Einrichtungen im Hinblick auf die Thrombozytengewinnung erläutert wird, ist es jedoch selbstverständlich möglich, die erfindungsgemäße Vorrichtungen in gleicher Weise auch auf die Gewinnung anderer Blutzellen, wie Leukozyten, z.B. Lymphozyten und Granulozyten, anzuwenden.

Für das zur Zellseparation, insbesondere für die Thrombozytengewinnung, oft benutzte Zweistufenverfahren wird in der ersten Stufe das thrombozytenreiche Plasma (PRP) von den schnell sedimentierenden Erythrozyten (RBC) getrennt, es werden dann in der nächsten Stufe die sich noch im Plasma befindlichen Thrombozyten (PLT) gewonnen (durch Zentrifugation eventuell durch Filtration).

Für den Thrombozytenfluß nach der ersten Stufe gilt:

(1) $\quad N_{PLT}=Q_{PRP} \cdot n_{PRP} \qquad N_{PLT}=$ (Thrombozyten)fluß [Teilchen/Zeit]

$Q_{PRP}=$ Fluß des (thrombozytenreichen) Plasmas [Volumen/Zeit]

$n_{PRP}=$ (Thrombozyten)dichte im PRP [Teilchen/Volumen],

Die runden Klammern in den Bezeichnungen sollen hierbei angeben, daß diese Gleichungen für verschiedene Zellsorten, wie z.B. Leukozyten, ebenfalls gelten.

Der Plasmafluß ergibt sich aus dem Volumenfluß $Q_{WB}$ und dem Hämatokritwert HK:

(2) $\qquad Q_{PRP}=Q_{WB} \cdot (1-HK)$

(3) $\qquad Q_{WB}=Q_{WB}(1-HK)+Q \cdot HK$

Die Gleichung (3) gilt für vollständige Sedimentation der RBC in der Zentrifuge, (1) wird damit:

(4) $\qquad N_{PLT}=Q_{WB} \cdot (1-HK) \cdot n_{PRP}$

Da die Sedimentationsgeschwindigkeit der Erythrozyten gegen die der Thrombozyten sehr groß ist, können die physikalischen Parameter der Zentrifuge ($Q_{WB}$, Radius, Drehzahl, Kammervolumen) so gewählt werden, daß die Erythrozyten sedimentiert sind, die Thrombozyten sich im wesentlichen noch in der anfänglichen Gleichverteilung n befinden. (Auf weitere Effekte, z.B. dynamisches Strömungsverhalten, Schwarmbehinderung der Zellen untereinande sowie das Hochtragen der sehr leichten Thrombozyten soll aus Verständnisgründen in diesem Fall nicht eingegangen werden).

Es ist dann:

$n_{PRP}=n_{WB} \qquad n_{WB}=$ (Thrombozyten)dichte im Vollblut (Teilchen/Volumen)

(5) $\qquad N_{PLT}=Q_{WB}(1-HK) \cdot n_{WB}$

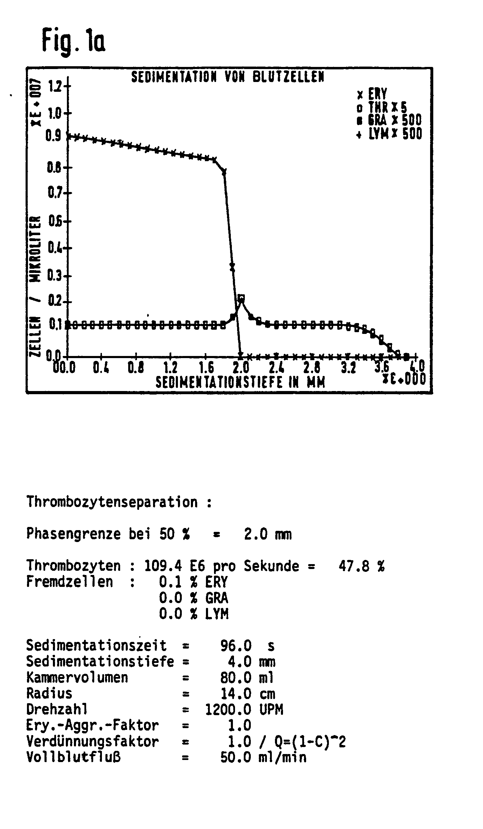

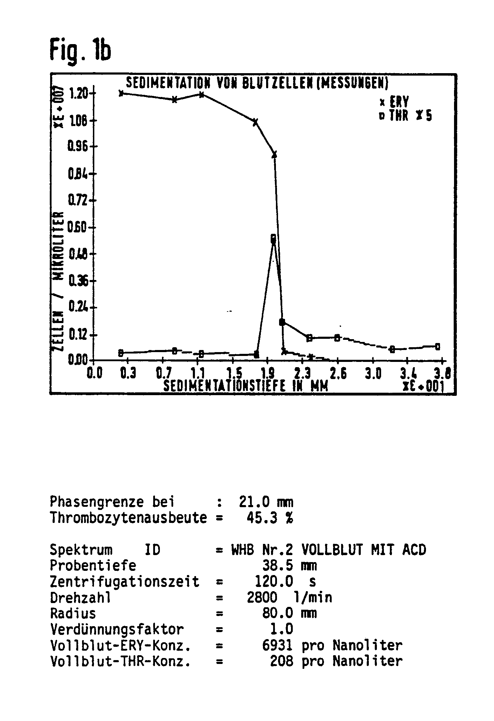

Es ist ersichtlich, daß bei diesem Verfahren die von den Erythrozyten eingeschlossenen Thrombozyten nicht zu gewinnen sind; dieser Verlustanteil ist proportional dem eingestelltem Hämatokritwert HK (vgl. Fig. 1).

Wird daher der Hämatokritwert in Gleichung (5) durch Hinzufügen einer Verdünnung verringert, so vergrößert sich der maximal erreichbare Thrombozytenfluß $N_{PLT}$ (bei gleichem Vollblutfluß $Q_{WB}$). Eine praktische Ausführung der durchzuführenden Verdünnung ist eine kontinuierliche Rückführung eines Teils des separierten thrombozytenarmen Plasmas (PPP). Dieses nach der zweiten Separationsstufe zur Verfügung stehende Plasma wird entweder zu dem Vollblut (WB) oder den Erythrozyten (RBC) zurückgeführt. Das bedeutet eine entsprechende Verringerung des Hämatokritwertes HK.

Gleichung (5) wird mit der Rückführung zu

(6)
$$N_{PLT} = [Q_{WB}(1-HK) + Q_{rück}] \cdot n_{WB}\left(\frac{Q_{WB}}{Q_{WB} + Q_{rück}}\right)$$

Werden die physikalischen Parameter der Zentrifuge so ausgelegt, daß eine Sedimentation der Erythrozyten trotz der Erhöhung des Zentrifugendurchsatzes

$$Q_Z = Q_{WB} + Q_{rück}$$

möglich ist, so wird als Verbesserung V bei der Thrombozytenausbeute:

(7)
$$V = \frac{[Q_{WB} \cdot (1-HK) + Q_{rück}] \cdot n_{WB} \cdot \left(\frac{Q_{WB}}{Q_{WB} + Q_{rück}}\right)}{Q_{WB} \cdot (1-HK) \cdot n_{WB}}$$

erhalten.

Wird das Plasma z.B. im Verhältnis 1:1 mit dem Vollblut zurückgeführt ($Q_{rück} = $ WB), so ergibt sich bei:

$$\left.\begin{array}{l} \text{HK 0,5 (Vollblut)} \\ Q_{rück} = Q_{WB} \end{array}\right\} \text{ mit Gleichung (7) V=1,5}$$

Bei noch höheren Verdünnungen ist als Grenzwert eine Verbesserung um maximal Faktor 2 möglich, d.h. es werden dann 100% PLT gewonnen.

Geschieht die Rückführung nicht in das Vollblut vor der Separationskammer, sondern in die Erythrozyten (RBC), die separiert in der ersten Stufe in der Separationskammer liegen, so kommt es bei gleicher Rückführungsmenge zu einem höheren Verbesserungsfaktor. Dieses bedeutet jedoch, daß eine zusätzliche Zuführung in die Separationskammer nötig ist.

Eine Erhöhung des Zentrifugendurchsatzs $Q_Z$

$$Q_Z = Q_{WB} + Q_{rück}$$

bedeutet natürlich gleichzeitig eine Verringerung der Sedimentationszeit, d.h. der Aufenthaltszeit eines Teilchens in der Zentrifuge. Eine hohe Verdünnung stößt deshalb an die technische Grenze der Zentrifugenleistung, d.h. die Aufenthaltsdauer wird kürzer als die benötigte Sedimentationszeit.

Eine Plasmarückführung 1:1 stellt hier einen Khompromiß dar, der auch technisch leicht zu realisieren ist. Die Aufenthaltszeit wird um den Faktor 2 verringert, gleichzeitig verringert sich aber die benötigte Sedimentationszeit für die Erythrozyten ebenfalls um≈2, so daß eine vollständige Sedimentation in der gleichen Zentrifuge mit gleichen physikalischen Bedingungen stattfindet.

Eine Verdünnung des zu separierenden Blutes durch die erfindungsgemäße Vorrichtung hat weiterhin den Vorteil, daß für eine hohe Zellausbeute der Hämatokritwert der zurückzuführenden Erythrozyten geringer sein kann als ohne diese Einrichtung. Aus physiologischer Sicht schadet ein hoher Hämatorkitwert der Lebensfähigkeit der Erythrozyten bzw. aller Zellen. Durch Kombinationen dieser beiden Vorteile kann man je nach Absicht Arbeitseinstellungen kombinieren:

schnell=hoher Hämatokritwert der rückgeführten Erythrozyten
schonend=geringer Hämatokritwert der rückgeführten Erythrozyten

Im allgemeinen wird abgetrenntes thrombozytenarmes Plasma im Verhältnis mit dem zu trennenden Vollblut in einer Menge im Bereich von 25%:75% bis 90%:10%, vorzugsweise im Bereich von 40%:60% bis 75%:25%, insbesondere 1:1 bis 2:1, d.h. etwa 50%:50% bis 66%:33% zurückgeführt. Bei Rückführung des abgetrennten zell- bzw. thrombozytenarmen Plasmas zu den im Separator befindlichen abgetrennten Erythrozyten beträgt das Verhältnis des zurückgeführten Plasmas zu den Erythrozyten etwa 62,5%:37,5% bis 95%:5%, vorzugsweise 70%:30% bis 87,5%:12,5% und beträgt insbesondere etwa 75%:25%.

Dieses Prinzip der Plasmarückführung ist ebenfalls für andere Zellsorten (Leukozyten (WBC), Granulozyten) anwendbar (hier z.B. mit Zusätzen von üblichen Sedimentationsbeschleunigern für die Erythrozyten, wie z.B. HES (Hydroxyethylstärke), Polysaccharide (Dextrane), Oxypolychelatine, Methylprednisolon oder Gemische derselben), da auch hier der Wirkungsgrad der Zellausbeute durch die von den Erythrozyten eingeschlossenen Anteile limitiert wird. Diese Sedimentationsbeschleuniger erzeugen dabei reversible Mikroaggregate, die besser sedimentieren. Eine Verdünnung durch Plasmarückführung führt hier zu den gleichen Verbesserungen wie in Gleichung (7). Die Gewinnung dieser Zellarten geschieht hier entweder als Zweistufenverfahren oder die Zellen werden nach abgeschlossener Sedimentation in der Trenngrenze gewonnen.

Da das Prinzip der Plasmarückführung erst im "eingefahrenen" Zustand, d.h. nach bereits genügend erfolgter Plasmaseparation nach Gleichung (7) funktioniert, bietet sich hierbei speziell die im System benutzte automatische Regelung der Phasengrenze (DE—PA 33 01 112.3) mittels der Plasmaförderpumpe an. Das Verfahren ist selbstverständlich auch mit anderen Verfahren zur Regelung der Phasengrenze kombinierbar.

Die vorliegende Erfindung ist nachfolgend weiter unter Bezugnahme auf die Zeichnungen erläutert. Es zeigen:

Fig. 1a eine graphische Darstellung des Sedimentationsprofils von Vollblut (Computersimulation analog den G1. 1—6),

Fig. 1b eine graphische Darstellung des Sedimentationsprofils von Vollblut analog Fig. 1a gemäß Messungen an Vollblut mit ACD (Antikoagulanz),

Fig. 2a eine graphische Darstellung des Sedimentationsprofils nach einem Verfahren mit Plasmarückführung, das mit der erfindungsgemäßen Vorrichtung durchgeführt wurde,

Fig. 2b eine graphische Darstellung des Sedimentationsprofils gemäß Messungen an Verdünnungen Vollblut: Plasma=1:1,

Fig. 3 eine graphische Darstellung des Sedimentationsprofils von Vollblut analog Fig. 1 unter Mitbetrachtung der Granulozyten und Lymphozyten,

Fig. 4 eine graphische Darstellung des Sedimentationsprofils von Vollblut unter Mitbetrachtung von Granulozyten und Lymphozyten nach einem Verfahren mit Plasmarückführung, das mit der erfindungsgemäßen Vorrichtung durchgeführt wurde,

Fig. 5a eine schematische Darstellung des Prinzips der Plasmarückführung außerhalb der Zentrifuge in das Vollblut,

Fig. 5b eine schematische Darstellung des Prinzips der Plasmarückführung außerhalb der Zentrifuge analog Fig. 5a, aber mit separater Pumpe (P),

Fig. 6a eine schematische Darstellung des Prinzips der Plasmarückführung außerhalb der Zentrifuge in die in der Zentrifuge befindlichen abgetrennten Erythrozyten,

Fig. 6b eine schematische Darstellung des Prinzips der Plasmarückführung analog Fig. 6a, aber mit separater Pumpe (P),

Fig. 7 eine Schnittansicht einer Separationskammer bei Plasmarückführung in die separierten Erythrozyten gemäß Fig. 6a und 6b, jedoch ohne Leitungen.

In Fig. 1a ist die Konzentration der Zellen als Funktion der radialen Sedimentationsstrecke, d.h. Kammertiefe, nach der ersten Separationsstufe dargestellt. Die in Fig. 1a unterhalb der Erythrozyten liegenden Thrombozyten (<2 mm) sind für die Zellgewinnung in der zweiten Stufe im wesentlichen verloren. Die sich oberhalb (>2 mm) der Erythrozyten befindenden Thrombozyten werden in der zweiten Separationsstufe mit einem prozentualen Anteil von 47,8 (vom Vollblut) gewonnen.

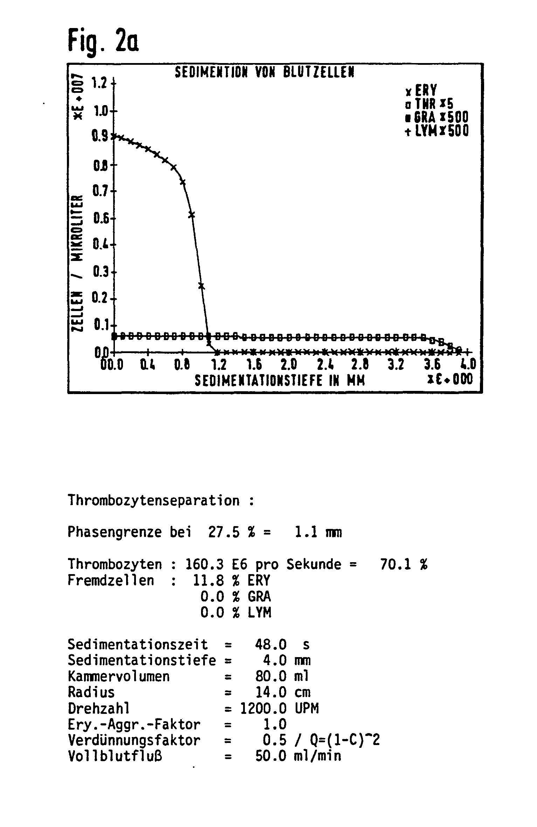

Aus Fig. 2a ist der erfindungsgemäß durch Plasmarückführung erzielbare Vorteil ersichtlich. Bei gleicher Kammer und gleichem Vollblutfluß (50 ml/min) wie gemäß dem in Fig. 1a angewandten Verfahren, aber unter Plasmarückführung, sind trotz der Plasmarückführung von 1:1 und der damit verbundenen Verkürzung der Aufenthaltszeit (1/2) die Erythrozyten fast vollständig sedimentiert.

Die Verbesserung der Thrombozytengewinnung auf 70,1% bedeutet einen Faktor von 1,47, was mit den theoretischen Ergebnissen nach Gleichung (7) gut übereinstimmt.

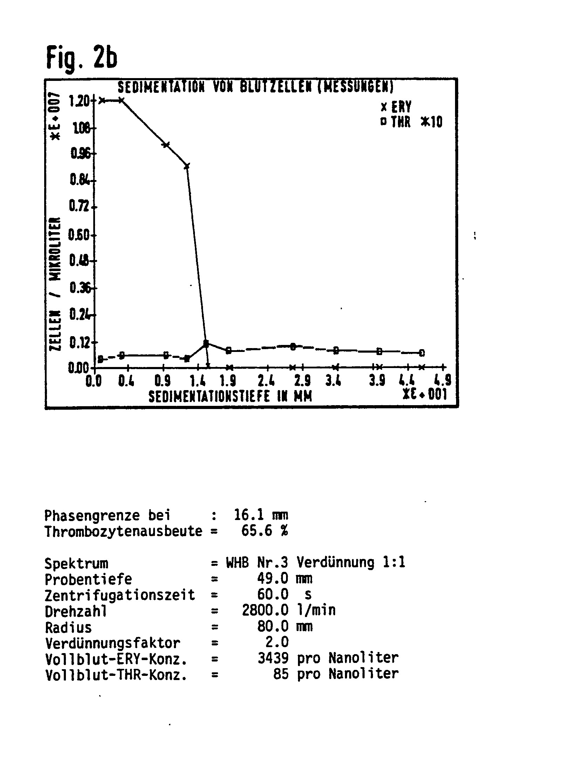

In Fig. 1b und 2b werden die Betrachtungen aus Fig. 1a und 2a durch Messungen bestätigt. Der prozentuale Anteil des unterhalb der Erythrozyten liegenden Zellanteils ist zahlenmässig etwas von dem in Fig. 1a und 2a verschieden, was mit der vereinfachten Betrachtung in Fig. 1a und 2a erklärt werden kann. Die Verbesserung der Zellanteile mit der Plasmarückführung ist jedoch qualitativ sehr gut zu sehen.

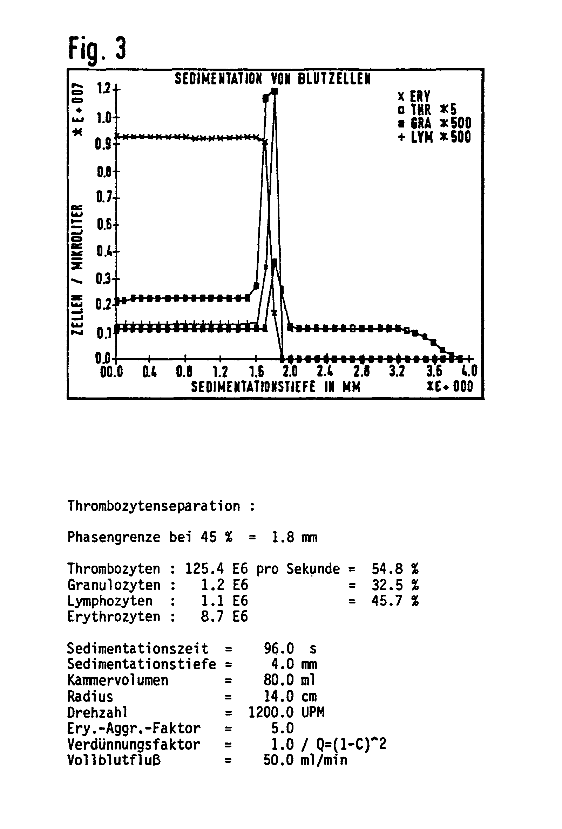

Gemäß der in Fig. 3 veranschaulichten graphischen Darstellung des Sedimentationsprofils von Vollblut wurden gegenüber Fig. 1 zusätzlich die Granulozyten und Lymphozyten mitbetrachtet. Außerdem wurde mit einem Sedimentationsbeschleuniger für die Erythrozyten (HES) gearbeitet. Die Zellen bilden auf den Erythrozyten eine Schicht und können hier entnommen werden.

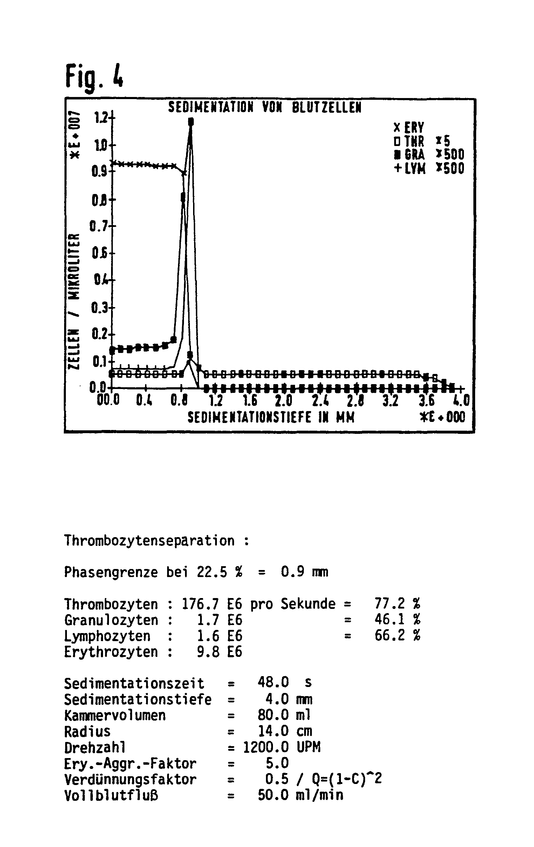

Fig. 4 veranschaulicht die erfindungsgemäß durch Plasmarückführung erzielbare Verbesserung gegenüber den in Fig. 3 dargestellten Ergebnissen, wobei mit gleicher Separationskammer und gleichem Vollblutfluß gearbeitet wurde. Für die Thrombozytengewinnung bedeutet die Plasmarückführung eine Verbesserung von 54,8% auf 77,2%. Die Verbesserung für die Zellgewinnung bezieht sich ebenfalls auf die übrigen im Vollblut befindlichen Blutzellen. Anstelle einer zweiten Sedimentationsstufe können hier die Zellen direkt an der Zellgrenze entnommen werden.

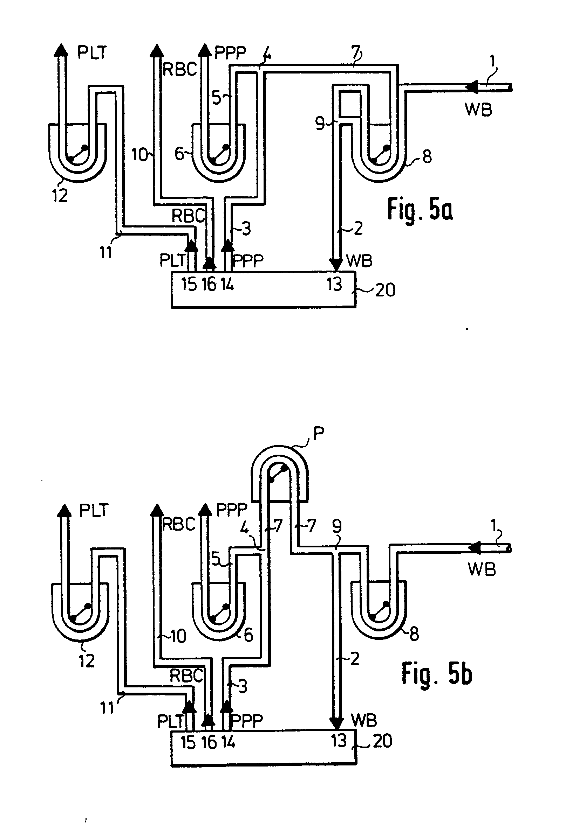

In Fig. 5a ist die Zentrifuge bzw. der Separator lediglich schematisch dargestellt, da das Verfahren mit jedem Separator, unabhängig von dessen Bauweise und unabhängig davon, ob eine oder mehrere Separationskammern vorliegen, durchgeführt werden kann, vorausgesetzt, daß die in Fig. 5 dargestellten Zuführungsleitungen und Abführungsleitungen vorliegen. Beispielsweise können die in der DE—A—28 45 399, DE—A—28 45 364, DE—A—28 21 057, DE—A—26 24 154, DE—A—28 21 055, DE—A—29 25 010, DE—A—26 12 988, US—A—43 30 080 und US—A—39 55 755 beschriebenen Separationseinrichtungen verwendet werden.

6

Mit 1 und 2 werden in Fig. 5 die Zuführungs- (bzw. Beschickungs-) leitungen für das Vollblut (WB) bezeichnet. Über die Leitung 3 wird das zell- bzw. thrombozytenarme Plasma (PPP) aus dem Separator ausgetragen. Bei 4 teilt sich diese Leitung 3 in eine Leitung 5, in der dieses zell- bzw. thrombozytenarme Plasma zu einer Schlauchpumpe (Plasmapumpe) 6 geführt und von dort ausgetragen bzw. zum Spender geführt wird, und eine Leitung 7, die zusammen mit de das Vollblut führenden Leitung 1 in eine Doppelschlauchpumpe 8 (die abhängig vom jeweiligen Schlauchquerschnitt auch unterschiedliche Mengen fördern kann) gelegt und stromab der Pumpe 8 bei der Verzweigung 9 mit der Leitung 1 vereinigt wird. Das mit dem rückgeführten Plasma versetzte Vollblut wird dann über die Leitung 2 in die Separationskammer geführt, wo es dem vorstehend beschriebenen, gegebenenfalls zweistufigen Separationsverfahren unterworfen wird. Über die Leitung 10 werden die Erythrozyten (RBC) und über die Leitung 11 und die Pumpe 12 die Thrombozyten (PLT) entfernt.

Die Pumpe 8 ist im Gegensatz zu den anderen Pumpen des Systems, d.h. Pumpen 6 und 12, nicht regelbar, sondern wird konstant betrieben und fördert konstant vorbestimmte Mengen.

Obgleich in der vorstehenden Fig. 5a das zell- bzw. thrombozytenarme Plasma dem Vollblut vor Erreichen der Separationskammer mittels der Doppelschlauchpumpe 8 zugeführt wird, is es jedoch auch möglich, eine separate, konstant betriebene Pumpe anzuwenden, wie das in Fig. 5b dargestellt wird. Die in Fig. 5b verwendeten Bezugszahlen entsprechen im wesentlichen den in Fig. 5a verwendeten, mit der Ausnahme, daß in Fig. 5b die konstant betriebene, vorbestimmte Mengen fördernde Pumpe mit P bezeichnet wird. Die Verfahrensweise entspricht der für Fig. 5a angegebenen; wie dort wird auch gemäß Fig. 5b das zurückgeführte Plasma (PPP) bei 9 mit dem zu trennenden Vollblut vereinigt.

Im Verfahren das mit der erfindungsgemäßen Vorrichtung durchgeführt werden kann, ist die Rückflußrate völlig unkritisch. Sie kann beliebig sein und kann knostant gehalten werden. Außerdem sind erfindungsgemäß Messungen von Eingangsgrößen nicht erforderlich.

Erfindungsgemäß läuft das System selbständig ein und nach dem Einlaufen stört die Rezirkulation des Plasmas die Separation nicht mehr, die Trenngrenze zwischen den Thrombozyten und den roten Blutkörperchen bleibt an der gleichen Stelle erhalten, wenn die Leistungsfähigkiet der Zentrifuge zur vollständigen Trennung des zusätzlichen Volumens ausreicht.

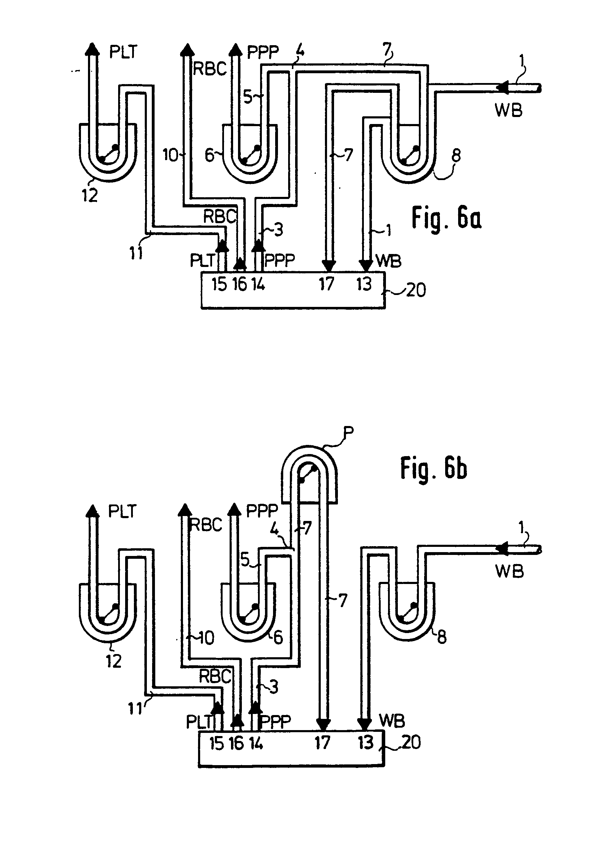

Analog wie in den Fig. 5a und 5b ist in Fig. 6a und 6b die Zentrifuge bzw. der Separator lediglich schematisch dargestellt. Von den Fig. 5a und 5b unterscheiden sich die Fig. 6a und 6b im wesentlichen darin, daß die Rückführung des abgetrennten Plasmas nicht in das Vollblut, sondern in die im Separator befindlichen abgetrennten Erythrozyten erfolgt. Für gleiche Teile sind in den Fig. 6a und 6b die gleichen Bezugszahlen wie in den Fig. 5a und 5b verwendet. So werden in Fig. 6a mit 1 und 2 die Zuführungs- (bzw. Beschickungs-)leitungen für das Vollblut (WB) bezeichnet, wobei das Vollblut durch die Öffnung 13 in die Zentrifuge bzw. den Separator 20 eingeführt wrid. Über die Leitung 3 wird das zell- bzw. thrombozytenarme Plasma (PPP) durch die Öffnung 14 aus dem Separator ausgetragen. Bei 4 teilt sich die Leitung 3 in eine Leitung 5, in der dieses zell- bzw. thrombozytenarme Plasma zu einer Schlauchpumpe (Plasmapumpe) 6 geführt und von dort ausgetragen bzw. zum Spender geführt wird, und eine Leitung 7, in der ein Teil des thrombozytenarmen Plasmas zunächst in eine Doppelschlauchpumpe 8, die abhängig vom jeweiligen Schlauchquerschnitt auch unterschiedliche Mengen fördern kann, geführt und dann bei Öffnung 17 in den Separator radial von außen eingeführt und mit den separierten Erythrozyten vereinigt wird. Durch die Öffnung 16 werden die Erythrozyten (RBC) aus dem Separator ausgetragen und dann über die Leitung 10 entfernt. Die Thrombozyten (PLT) werden aus dem Separator durch die Öffnung 15 ausgetragen und dann über die Leitung 11 und die Pumpe 12 entfernt.

Die Pumpe 8 ist im Gegensatz zu den anderen Pumpen des Systems, d.h. den Pumpen 6 und 12, nicht regelbar, sondern wird konstant betrieben und fördert konstant vorbestimmte Mengen.

Obgleich in der vorstehenden Fig. 6a das zell- bzw. thrombozytenarme Plasma mittels der Doppelschlauchpumpe 8 dem Separator durch Öffnung 17 zugeführt wird, ist es jedoch auch möglich, eine separate, konstant betriebene Pumpe anzuwenden, wie das in Fig. 6b dargestellt wird. Die in Fig. 6b verwendeten Bezugszahlen entsprechen im wesentlichen den in Fig. 6a verwendeten Bezugszahlen, mit der Ausnahme, daß in Fig. 6b die konstant betriebene, vorbestimmte Mengen fördernde Pumpe mit P bezeichnet wird. Die Verfahrensweise entspricht der für Fig. 6a angegebenen. Wie dort wird auch gemäß Fig. 6b das zurückgeführte Plasma (PPP) durch die Öffnung 17 in den Teil des Separators eingeführt, in dem sich die bereits abgetrennten Erythrozyten befinden.

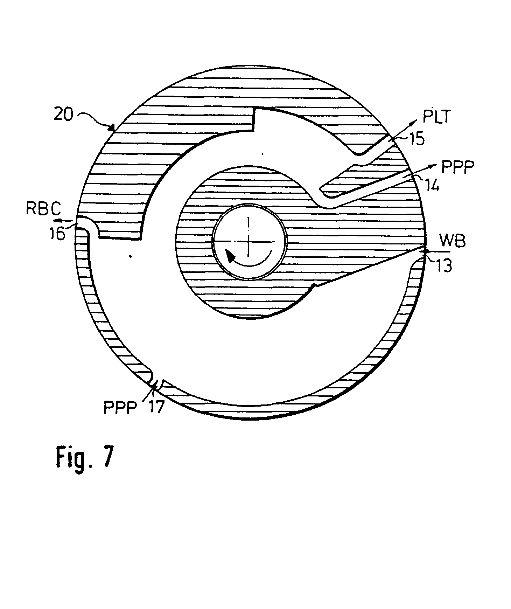

Fig. 7 veranschaulicht einen Schnitt durch eine Separationskammer 20, wobei entsprechend der erfindungsgemäßen Verfahrensweise abgetrenntes thrombozytenarmes PLasma zu den separierten Erythrozyten in der Separationskammer zurückgeführt wird. Durch die Öffnung 13 wird das zu trennende Vollblut in die Separationskammer eingeführt, wo sich zunächst die Erythrozyten abtrennen und dann in den Segmenten mit geringerem Radius die zweite Stufe der Trennung, d.h. die Trennung des thrombozytenreichen Plasmas in die Thrombozyten und das thrombozytenarme Plasma, erfolgt. Durch die Öffnung 14 wird das thrombozytenarme Plasma und durch die Öffnung 15 wird die Thrombozytenfraktion entfernt. Das wie in Fig. 6a und 6b beschriebene- erfindungsgemäß zurückgeführte Plasma gelangt radial von außen durch die Öffnung 17 in die Separationskammer zu den separierten Erythrozyten. Die separierten Erythrozyten werden durch die Öffnung 16 entfernt. Wie in Fig. 6a und 6b dargestellt, sind auch hier alle Öffnungen in der Separationskammer entsprechend mit Zuführungs- bzw. Abführungsleitungen verbunden. In gleicher Weise ist die Öffnung 17 mit der Zuführungsleitung 7 und der konstant betriebenen

7

# EP 0 155 684 B1

Pumpe 8 bzw. P verbunden.

Anstelle einer Verdünnung des gesamten Vollblutes findet hier gemäß den Fig. 6a, 6b und 7 nur eine Verdünnung der Erythrozyten statt, wodurch bei gleicher Menge an rückgeführtem Plasma der Verbesserungsfaktor V (siehe Gleichung (7)) gegenüber dem Faktor, der gemäß dem in Fig. 5a und 5b veranschaulichten Verfahren erhalten wird, größer wird.

Der Bereich um die Öffnung 17 kann gegebenenfalls zusätzlich als Mischkammer ausgelegt werden.

Die Gewinnung der Leukozyten an sich kann in üblicher Weise durchgeführt werden, wobei jedoch erfindungsgemäß, wie vorstehend beschrieben, zellarmes Plasma zurückgeführt wird. Die vorstehend für die Thrombozytengewinnung gemachten Angaben sind in gleicher Weise, jedoch entsprechend der Leukozytengewinnung abgewandelt, auf die Leukozytengewinnung anwendbar.

So kann die Leukozytengewinnung im wesentlichen so durchgeführt werden, daß man zunächst aus dem Vollblut die Erythrozyten mit Hilfe von üblichen Sedimentationsbeschleunigern (wie z.B. HES) sedimentiert. Die Leukozyten können sich dann auf den Erythrozyten als Schicht ablagern und von dort direkt an der Trenngrenze abgezogen werden. Das zellarme (leukozytenarme, thrombozytenreiche) Plasma kann dann, wie vorstehend beschrieben, entfernt werden und/oder gegebenenfalls in einer weiteren Trennstufe in Thrombozyten und thrombozytenarmes (zellarmes) Plasma aufgetrennt werden. Das zellarme Plasma wird anschließend, wie vorstehend angegeben, teilweise zurückgeführt.

Es ist jedoch auch möglich, so zu verfahren, daß man, wie vorstehend beschrieben, aus dem Vollblut mittels üblichen Sedimentationsbeschleunigern zunächst die Erythrozyten abtrennt, in der nächsten Trennstufe das zellreichet Plasma in die Leukozyten und zellarmes (leukozytenarmes) Plasma trennt und dann, wie beschrieben, erfindungsgemäß einen Teil des zellarmen Plasmas zurückführt. Gegebenenfalls kann in einer weiteren Trennstufe das zellarme Plasma in die Thrombozyten und thrombozytenarmes Plasma aufgetrennt werden und dann wiederum erfindungsgemäß ein Teil des zellarmen (thrombozytenarmen) Plasmas zurückgeführt werden.

## Patentansprüche

1. Separationseinrichtung zur Durchführung eines Verfahrens zur Trennung von Blut, insbesondere zur Gewinnung von Thrombozyten, Leukozyten und/oder Plasma, umfassend einen Separator (20) mit einer oder mehreren miteinander verbundenen Trennkammern sowie ein Schlauchsystem, das eine Zuführungsleitung (1, 2) für die Zufuhr des zu trennenden Vollblutes (WB) zum Separator (20) sowie Abführungsleitungen jeweils für das getrennte Austragen der Erythrozytenfraktion (RBC), des zellarmen Plasmas (PPP), der Thrombozytenfraktion (PLT) und/oder gegebenenfalls von Leukozytenfraktionen und eine vom Separator (20) ausgangsseitig in der Abführungsleitung für das zellarme Plasma (PPP) angeordnete Verzweigungsleitung (7) zur Rückführung von zellarmen Plasma (PPP), wobei die Zu- und Abführungsleitungen des Schlauchsystems mit Pumpem ausgestattet sind, umfaßt, dadurch gekennzeichnet, daß die ausgangsseitig am Separator (20) angeordnete Verzweigungsleitung (7) vor der Plasmapumpe (6) abgeführt ist und in der Verzweigungsleitung (7) eme Pumpe (8 bzw. P) vorgesehen ist, die eine vorbestimmte Plasmamenge fördert.

2. Separationseinrichtung nach Anspruch 1, dadurch gekennzeichnet, daß in den Zu- und Abführungsleitungen regelbare Pumpen vorliegen.

3. Separationseinrichtung nach Anspruch 1 oder 2, dadurch gekennzeichnet, daß die Verzweigungsleitung (7) mit der Zuführungsleitung (1) für das zu trennende Vollblut stromab der Blutpumpe (8) verbunden ist.

4. Separationseinrichtung nach Anspruch 1 oder 2, dadurch gekennzeichnet, daß die Verzweigungsleitung (7) mit dem Teil der Separationskammer verbunden ist, in dem sich separierte Erythrozyten befinden.

## Revendications

1. Dispositif de séparation pour la mise en oeuvre d'un procédé de séparation du sang, en particulier pour la récupération de plaquettes, de leucocytes et/ou de plasma, comprenant un séparateur (20) avec une ou plusieurs chambres de séparation, reliées les unes aux autres, ainsi qu'un système de tuyaux souples, lequel englobe une conduite d'amenée (1, 2), destinée à amener au séparateur (20) le sang total (WB) à séparer, ainsi que des conduites d'évacuation, chacune étant destinée à évacuer séparément la fraction érythocytaire (RBC), le plasma pauvre en éléments figurés (PPP), la fraction plaquettaire (PLT) et/ou éventuellement des fractions leucocytaires, ainsi qu'une conduite dérivée (7), disposée côté sortie du séparateur (20) dans la conduite d'évacuation du plasma pauvre en éléments figurés (PPP), et destinée à renvoyer le plasma pauvre en éléments figurés (PPP), les conduites d'amenée et d'évacuation du système de tuyaux souples étant pourvues de pompes, caractérisé en ce que la conduite dérivée (7), disposée côté sortie du séparateur (20), passe en amont de la pompe à plasma (6) et que, dans la conduite dérivée (7), une pompe (8 ou P) est prévue, qui refoule un débit prédéfini de plasma.

2. Dispositif de séparation selon la revendication 1, caractérisé en ce que des pompes réglables sont disposées dans les conduites d'amenée et d'évacuation.

3. Dispositif de séparation selon la revendication 1 ou 2, caractérisé en ce que la conduite dérivée (7)

8

## EP 0 155 684 B1

est reliée en aval de la pompe à sang à la conduite d'amenée (1) du sang total à séparer.

4. Dispositif de séparation selon la revendication 1 ou 2, caractérisé en ce que la conduite dérivée (7) est reliée à la partie de la chambre de séparation dans laquelle se trouvent les érythrocytes séparés.

**Claims**

1. Separating device for carrying out a method of separating blood, in particular for recovering thrombocytes, leucocytes and/or plasma, comprising a separator (20) having one or several separating chambers being connected with each other as well as a tubing system which includes a supply line (1, 2) for the supply of the whole blood (WB) to be separated to the separator (20) as well as discharge lines for the separate discharge of each of the erythrocyte fraction (RBC), the cell-poor plasma (PPP), the thrombocyte fraction (PLT) and/or optionally of leucocyte fractions, and a branch line (7) disposed at the outlet of the separator in the discharge line for the cell-poor plasma (PPP) for returning clel-poor plasma (PPP), whereby the supply and discharge lines of the tubing system are provided with pumps, characterized in that the branch line (7) arranged at the outlet of the separator (20) is branched off stream up the pump (6) and that in the branch line (7) a pump (8, resp. 9) is provided which conveys a predetermined amount of plasma.

2. Separating device according to claim 1, characterized in that regulatable pumps are provided.

3. Separating device according to claim 1 or 2, characterized in that the branch line (7) is connected to the supply line (1) for the whole blood to be separated downstream of the blood pump (8).

4. Separating device according to claim 1 or 2, characterized in that said branch line (7) is connected to the part of the separation chamber in which separated erythrocytes are disposed.

## Fig. 1a

SEDIMENTATION VON BLUTZELLEN

x ERY
□ THR X 5
■ GRA X 500
+ LYM X 500

Thrombozytenseparation :

Phasengrenze bei 50 %   =   2.0 mm

Thrombozyten : 109.4 E6 pro Sekunde =   47.8 %
Fremdzellen  :   0.1 % ERY
                 0.0 % GRA
                 0.0 % LYM

Sedimentationszeit   =     96.0  s
Sedimentationstiefe  =      4.0  mm
Kammervolumen         =     80.0  ml
Radius                =     14.0  cm
Drehzahl              =   1200.0  UPM
Ery.-Aggr.-Faktor     =      1.0
Verdünnungsfaktor     =      1.0 / Q=(1-C)^2
Vollblutfluß          =     50.0  ml/min

1

## Fig. 1b

SEDIMENTATION VON BLUTZELLEN (MESSUNGEN)

```
Phasengrenze bei       :   21.0 mm
Thrombozytenausbeute   =   45.3 %

Spektrum     ID        = WHB Nr.2 VOLLBLUT MIT ACD
Probentiefe                 38.5 mm
Zentrifugationszeit    =    120.0  s
Drehzahl               =    2800  1/min
Radius                 =     80.0 mm
Verdünnungsfaktor      =      1.0
Vollblut-ERY-Konz.     =     6931 pro Nanoliter
Vollblut-THR-Konz.     =      208 pro Nanoliter
```

2

# Fig. 2a

Thrombozytenseparation :

Phasengrenze bei  27.5 % =   1.1 mm

Thrombozyten : 160.3 E6 pro Sekunde =   70.1 %
Fremdzellen  :  11.8 % ERY
                 0.0 % GRA
                 0.0 % LYM

Sedimentationszeit   =    48.0  s
Sedimentationstiefe  =     4.0  mm
Kammervolumen        =    80.0  ml
Radius               =    14.0  cm
Drehzahl             =  1200.0  UPM
Ery.-Aggr.-Faktor    =     1.0
Verdünnungsfaktor    =     0.5 / Q=(1-C)^2
Vollblutfluß         =    50.0  ml/min

# Fig. 2b

SEDIMENTATION VON BLUTZELLEN (MESSUNGEN)

x ERY
□ THR *10

ZELLEN / MIKROLITER *E+007

SEDIMENTATIONSTIEFE IN MM     *E+001

```
Phasengrenze bei        :   16.1 mm
Thrombozytenausbeute =      65.6 %

Spektrum               =  WHB Nr.3 Verdünnung 1:1
Probentiefe            =      49.0 mm
Zentrifugationszeit    =      60.0  s
Drehzahl               =    2800.0 1/min
Radius                 =      80.0 mm
Verdünnungsfaktor      =       2.0
Vollblut-ERY-Konz.     =      3439 pro Nanoliter
Vollblut-THR-Konz.     =        85 pro Nanoliter
```

## Fig. 3

Thrombozytenseparation :

Phasengrenze bei 45 % = 1.8 mm

| Thrombozyten : | 125.4 E6 pro Sekunde = | 54.8 % |
|---|---|---|
| Granulozyten : | 1.2 E6 | = 32.5 % |
| Lymphozyten : | 1.1 E6 | = 45.7 % |
| Erythrozyten : | 8.7 E6 | |

| Sedimentationszeit | = | 96.0 s |
|---|---|---|
| Sedimentationstiefe | = | 4.0 mm |
| Kammervolumen | = | 80.0 ml |
| Radius | = | 14.0 cm |
| Drehzahl | = | 1200.0 UPM |
| Ery.-Aggr.-Faktor | = | 5.0 |
| Verdünnungsfaktor | = | $1.0 / Q=(1-C)^2$ |
| Vollblutfluß | = | 50.0 ml/min |

# Fig. 4

Thrombozytenseparation :

Phasengrenze bei 22.5 %  =  0.9 mm

Thrombozyten : 176.7 E6 pro Sekunde =   77.2 %
Granulozyten :   1.7 E6               =   46.1 %
Lymphozyten  :   1.6 E6               =   66.2 %
Erythrozyten :   9.8 E6

Sedimentationszeit    =   48.0  s
Sedimentationstiefe   =    4.0  mm
Kammervolumen         =   80.0  ml
Radius                =   14.0  cm
Drehzahl              = 1200.0  UPM
Ery.-Aggr.-Faktor     =    5.0
Verdünnungsfaktor     =    0.5 / $Q=(1-C)^2$
Vollblutfluß          =   50.0  ml/min

Fig. 5a

Fig. 5b

Fig. 6a

Fig. 6b

Fig. 7